# EUROPEAN PATENT SPECIFICATION

(11) **EP 0 978 255 B1**
(45) Date of publication and mention of the grant of the patent: **13.12.2006**
(21) Application number: 99115042.6
(22) Date of filing: 03.08.1999
(51) Int. Cl.: A61B 5/00, A61B 5/04, A61B 5/0432

(54) **Ambulatory recorder having a real time processor and non-real time processor**
Gerät zum ambulanten Registrieren mit Echtzeit und Nicht-Echtzeit Prozessor
Appareil d'enregistrement ambulatoire avec système de traitment en temps réel et en temps non- réel

(30) Priority: 06.08.1998 US 130148
(43) Date of publication of application: 09.02.2000
(73) Proprietor: MEDTRONIC, INC., Minneapolis, Minnesota 55432-5604 (US)
(72) Inventor: Pedersen, Henrik E., 2760 Maaloev (DK); Williams, Malcolm G.S., 11628 Stockholm (SE); Minoz, Alain, 16738 Bromma (SE); Butler, Arch W., St. Louis Park, Minnesota 55416 (US)
(74) Representative: Hughes, Andrea Michelle

(56) References cited:
- EP-A- 0 512 667
- EP-A- 0 519 137
- WO-A-97/17012
- DE-A- 19 715 678
- US-A- 4 748 562
- US-A- 5 086 778

## Description

The present invention relates to ambulatory recording, for medical and especially for diagnostic purposes, and particularly to an ambulatory recorder having a real time processor and non-real time processor.

Various physiologic signals are often recorded and analyzed. These signals may include digestive pH, various digestive motility and pressure signals, EEG and EMG, to list only a few.

Typically, physicians require the concurrent recording of a variety of physiologic signals. For example, gastric pH is often collected at the same time as pressure. Through the concurrent collection of various parameters the physician may better understand the patient's condition.

Ambulatory recording and recorders are widely used to collect such data. Such devices include the Digitrapper Mk III^{™} ambulatory recorder from Synectics Medical AB, the GastroScan II^{™} from Medical Instruments Corporation, and the SuperLogger^{™} from Sandhill Scientific. These types of devices make it possible for patients to remain at home, or at the least be ambulant in a hospital setting while physiological data is recorded. Typically the devices comprise a lightweight recorder in which the desired physiological data signals are temporarily stored and later downloaded for future analysis.

Many types of physiological data may be recorded, including ECG (Electrocardiogram), EEG (Electroencephalogram) or pH and pressure (Motility) in the gastrointestinal tract. Preferably such a recorder should be able to record among a programmable number of channels at a variety of programmable frequencies.

EP 0512667 A1 describes a battery powered ambulatory ECG recorder comprising a sensor, a processor having a RAM and a hard disk wherein during a programming phase the processor is being configured to receive programming information. During a recording mode the processor is interrupt controlled and powered up at predetermined time periods to acquire new data and to store said data in said RAM, only when the RAM is nearly full the hard disk is activated and the content of the RAM is written to the hard disk. During a download phase, when the recording session is concluded, the disk drive is isolated from the processor and the data is transferred from the hard disk to a data processing system without involvement of the processor. EP 0356603 A1 describes a method for ambulatory recording and an ambulatory recorder.

Among the problems with current recorders, however, is that of energy usage. Such recorders, because they must be ambulatory, are battery powered. Thus an ambulatory medical recorder must minimize energy usage while performing almost constant sampling across a variable number of channels at one or more frequencies.

Accordingly, the present invention provides an ambulatory medical data recorder for recording at least one physiological signal sensed in a patient by a sensor, comprising a housing adapted to be worn or carried by a patient, the recorder containing:
a sensor coupled to said housing, for receiving the at least one sensed physiological signal; and the housing containing
at least one battery for providing a source of electrical current;
a first real time processor operably connected to the sensor input and powered by the at least one battery; and
a second non-real time processor operably connected to the first data processor and powered by the at least one battery;
wherein during a first programming phase said first processor is off and said second processor is powered and configured to receive programming information;
during a second sample data phase said first processor is intermittently powered on and awake at defined predetermined time periods during which said first processor is configured to receive, at least one sensed physiological signal from the sensor input and perform at least a first process thereon to yield a first data set and store same in a first memory
and the second processor is awake in regular intervals during which said second processor is configured to receive, the first data set from the first processor, and to store same in a second memory, and wherein
during a third interrogation and download data phase said first processor is off and said second processor is powered; and
during a fourth analysis phase both the first and second processors are powered down.

In a preferred embodiment of the invention, the first processor has means for programming the first processor to process a programmed number of physiologic parameters from the patient.

The real time processor preferably handles sampling functions while the non real time processor handles other functions, such as high processing operations associated with multitasking, graphical user interface, floating point calculation, Infra Red communication and long term memory storage. In particular, a second processor is primarily provided to operate a Windows CE operating system as well as one or more embedded applications. Through this design power is only consumed by those components essential to the device operation. That is only the real time (sampling) processor is operating while sampling is occurring. The non real time (operating system) processor only operates as needed. These as needed times include logging the sampled data into long term memory, re-configure system settings such as sampling frequency or channels to be sampled. In this manner an ambulatory medical recorder may be fashioned which has minimal power consumption while still offering many features.

Preferred embodiments will now be described, by way of example only, with reference to the accompanying drawings.
FIG. 1A depicts an ambulatory recorder according to the present invention.
FIG. 1B illustrates a further manner in which the recorder 1 may also have an infra red data communication link made with a host PC.
FIG. 2 is a block diagram of the data recording system shown in FIG. 1B.
FIG. 3 depicts the basic operational steps of a recorder according to the present invention.
FIG. 4 illustrates, over time, the relative periods during which the real time processor 21 and non-real time processor 22 are awake or in operation.
FIG. 5 is a back view of the recorder.
FIG. 6 is a side view of the recorder.

The FIGS. are not necessarily to scale.

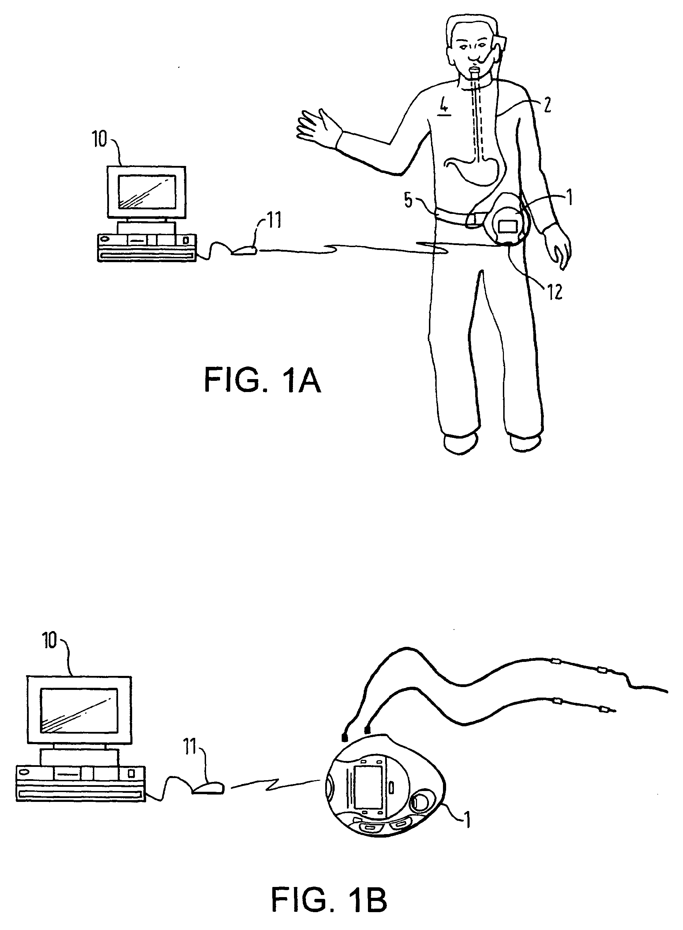

FIG. 1A depicts an ambulatory recorder of the present invention. As seen, ambulatory recorder 1 of the present invention may be carried by a patient. In the preferred embodiment, the recorder may be either carried through a mounting in the back of the recorder enclosure which fastens to a patient's belt 5, or the same mounting may be coupled to be carried using a shoulder harness (not shown). As seen, the recorder is coupled to the patient 4 through one or more sensing catheters 2. Sensing catheters may be positioned in any area of the patient's body, from which data is to be sensed, including the esophagus, as depicted in this FIG. It should be noted, the ambulatory recorder of the present invention may be used to collect many or various types of data including gastrointestinal (including pH and pressure), neurological, as well as neuromuscular, EEG or EMG data.

Among the various sensing catheters which may be coupled to the device are manometry catheters and pH testing catheters, including the Synectics Medical AB. Stockholm, Sweden, Model G 91-9 series of Multi use pH catheters; Synectics Medical AB Model G 91-2 series of Multi use pH catheters with perfusion port; or the Zinectics Inc., Salt Lake City, Utah, disposable 24 pH catheter Model series G 91-6 or G91-7. While a single catheter 2 is shown depicted in this figure, the recorder further permits two separate sensors to be coupled to the device, as seen in FIG. 1B.

As further seen in this figure, the recorder may also communicate with a host PC 10 via an infra red data link facility through an IRDA connection 11, for example, a JETEYE ESI-57680 available from Extended Systems, Inc., Boise, Idaho, which connects with the recorder using the Infra Red Data Association 1.1 Connection Protocol. As seen, the infra red data connection makes a link to infra red port 12 on the recorder.

FIG. 1B illustrates a further manner in which the recorder 1 may also have an infra red data communication link made with a host PC. In particular, the infra red data communication link may be further made when the recorder is not worn by the patient. As discussed in more detail below, one of the advantages of the preferred embodiment is that the infra red data components and recorder case permit such a link to be made when the device is worn as shown in FIG 1A as well as if the device is merely removed from the patient and positioned in proximity to mouse 11.

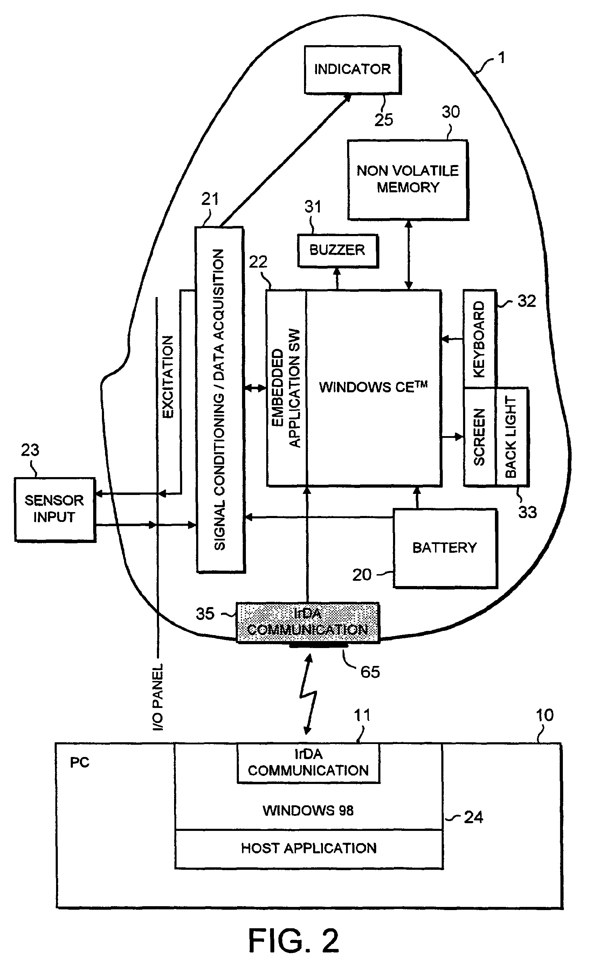

FIG. 2 is a block diagram of the data recording system shown in FIG. 1B. As seen, recorder 1 features a battery 20 which is coupled to the signal conditioning/data acquisition block that is driven by a real time processor 21. The battery is coupled as well to a non-real time processor 22 that runs the application. As disclosed in more detail below, real time processor 21 is a lower power processor which is used to sample data which is received from sensor input 23 by a sensor attached thereto (not shown in this FIG.).

Sampling is achieved through the signal conditioning providing an excitation to the sensor coupled to sensor input 23. Such excitation voltage is often used to power and, thus, permits sensing to occur in a variety of different types of sensors, including pressure sensors, as is well known in the art. The sampling and sensing controls are provided by the real time processor 21. Real time processor also drives an LED indicator 24 to show the system is running even when the screen is off.

As further seen, this processor is coupled to a second non-real time processor 22. Second processor 22 is provided primarily to perform those high processing operations associated with multitasking, graphical user interface, floating point calculation, Infra Red communication and long term memory storage. In particular, second processor is primarily provided to operate a Windows CE operating system as well as one or more embedded applications, as depicted. As further seen, this processor is coupled to audible buzzer 31 as well as keyboard controls 32, a screen 33 and non-volatile memory 30. Non-volatile memory provides a long term memory for the device such that data can be recorded and preserved even if power is lost. In the preferred embodiment, the keyboard controls processes a series of four push buttons, each of which provides one or more different types of system inputs, as provided by the Windows CE^{™} operating system, available from Microsoft Corporation, Redmond, Washington.

As further seen in this figure, the recorder features an infra red port 35 to communicate with the host PC. As depicted in FIG. 1B, the infra red connection permits the recorder 1 to receive and exchange data with host PC 10. The host PC, as seen, includes both a Windows 98^{™} operating system available from Microsoft Corporation, Redmond, Washington, as well as one or more host applications. Host applications permit the treatment of the recorded values and help for diagnostics.

In the preferred embodiment the real time processor is the model PIC16LC67 from Microchip Technology Inc., Chandler, Arizona; the non real time processor is the model ElanSC400 from Advanced Micro Devices, Inc., Sunnyvale, California; and the non-volatile memory is the model Minicard AMMCL004AWP from Advanced Micro Devices, Inc., Sunnyvale, California.

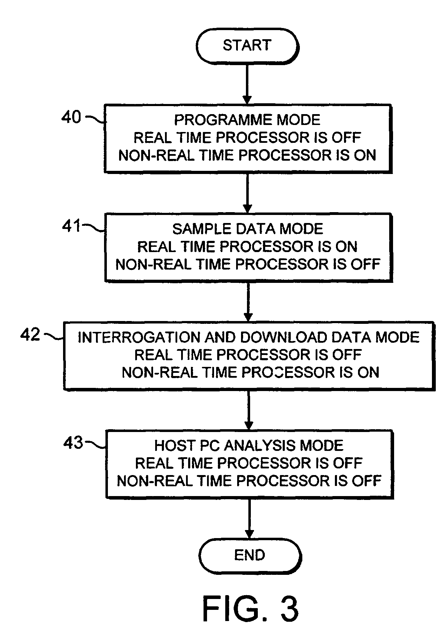

FIG. 3 depicts the basic operational steps of the present invention. In particular, this FIG. depicts the advantage and utility of the dual processor architecture which is used in the data recorder 1. That is, the data recorder is able to have only a particular and specialized processor powered during those times at which that type of processor operation is required.

As depicted, the system operational steps comprise essentially four time periods. In the first time period device programming 40 is performed. During this phase the host PC has an infra red data connection provided to recorder 1 and the non-real time processor 22 is powered. Through this connection the application is loaded into the recorder such that the type of catheter coupled to the device may be provided to the recorder, the number of and frequency upon which data is to be recorded is provided, the type of excitation voltages and patterns necessary for such catheters is also provided.

As further seen, during the step of programming, the real time processor 21 is turned off and the device is not collecting data. Next, the system enters into a recording data mode, during this time the non-real time processor 22 is powered down, i.e. turned off and only the real time processor 21 is activated. In particular, and as more thoroughly disclosed below, this real time processor is only powered on and awake at defined predetermined time periods during which data is to be collected. Thus, during those times in which no sampling is to occur, processor 21 is only powered upon request. In regular intervals and for a very short period of time the non-real time processor 22 is awake to collect a block of data and to save it into non-volatile memory 30. These sleep steps permit the device to draw a minimal amount of current and thus draw a larger amount of data over the life of its battery.

Next, after the desired amount of data sampling has occurred, the device enters into an interrogation and download data mode 42. This mode is entered into by again making an infra red connection with the host PC, as described above. At this time the real time processor 21 is powered down and the non-real time processor 22 is powered up. However, during this time, the memory contents are transferred from the recorder to the host PC. Finally, the system enters into a host PC analysis mode 43. In this mode data downloaded from the recorder is analyzed and/or manipulated by the host PC in any suitable manner to permit the physician to extract that information which is desired.

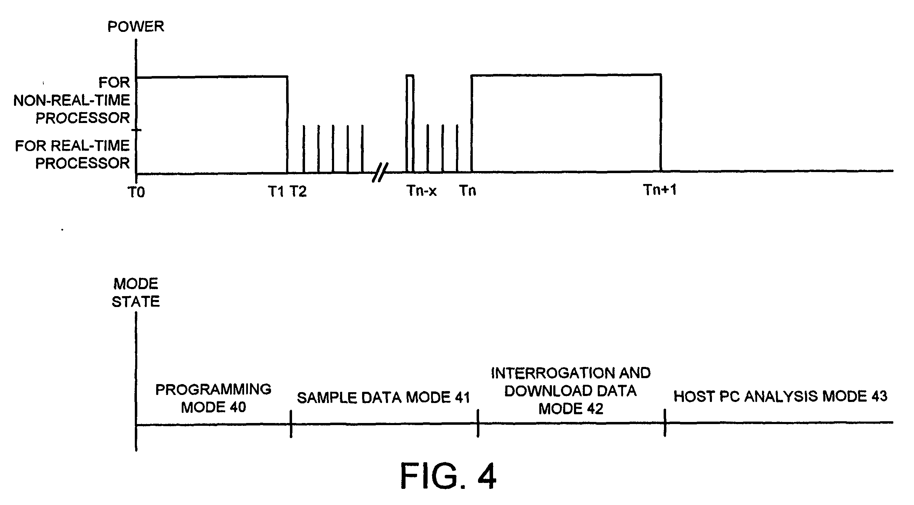

FIG. 4 illustrates, over time, the relative periods during which the real time processor 21 and non-real time processor 22 are awake or in operation. As seen, from time T0 to time T1, the non-real time processor 22 is in operation and, effectively, the device is in the programmer mode 40, described above. At this time, due to the current required to operate this processor, the overall power consumed by the power from the battery is at a power corresponding to Pnon-real. Next, the device enters the sample data mode 41 during which time the non-real time processor 22 is turned off and the real time processor 21 is intermittently and briefly turned on. As seen, this is depicted as individual spikes at times T2 - Tn. Moreover, as seen, the magnitude of the individual spikes corresponding with the awakening of the real time processor 21 is of less magnitude than the number of the non-real time processor 22, i.e. Preal is less than P non-real. As further seen, during sample data mode 41 the recorder would periodically awaken the non-real time processor, typically to transfer data captured in the volatile memory of the real time processor to the non-volatile memory of the recorder. Such an event is depicted here at Tn-x. Once sampling is completed the device would enter into an interrogation and download mode 42 during which time the non-real time processor 22 is again awakened and again the corresponding current drain escalated.

Finally, the system may enter into the host PC analysis mode 43 during which time the recorder itself may be completely turned off and no power is consumed, as seen above.

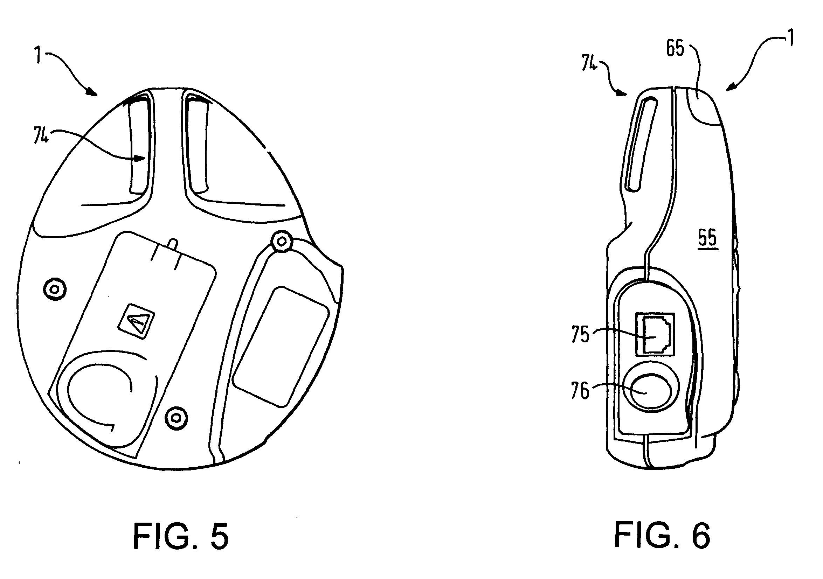

FIG. 5 is a back view of the recorder. As seen, recorder 1 features a belt loop 74 which may be used to mount the recorder to a patient using either the patient's belt or the shoulder strap.

FIG. 6 is a side view of recorder 1. As further seen in this view, housing 55 features a pair of sensor inputs 75 and 76. In the preferred embodiment, input 75 is for a pH catheter while input 76 is for a pressure measuring catheter.

## Claims

1. An ambulatory medical data recorder for recording at least one physiological signal sensed in a patient by a sensor, comprising a housing (55) adapted to be worn or carried by a patient, the recorder (55) containing:
a sensor (2, 23) coupled to said housing, for receiving the at least one sensed physiological signal; and the housing containing
at least one battery (20) for providing a source of electrical current;
a first real time processor (21) operably connected to the sensor input (23) and powered by the at least one battery; and
a second non-real time processor (22) operably connected to the first data processor and powered by the at least one battery;
wherein during a first programming phase said first processor is off and said second processor is powered and configured to receive programming information;
during a second sample data phase, said first processor is intermittently powered on and awake at defined predetermined time periods during which said first processor is configured to receive
at least one sensed physiological signal from the sensor input and perform at least a first process thereon to yield a first data set and store same in a first memory and the second processor is awake in regular intervals during which said second processor is configured to receiver the first data set from the first processor, and to store same in a second memory, and wherein
during a third interrogation and download data phase said first processor is off and said second processor is powered; and
during a fourth analysis phase both the first and second processors are powered down.

2. An ambulatory recorder according to claim 1 wherein the first processor comprises means (21) for sampling the sensed physiologic parameter.

3. An ambulatory recorder according to claim 2 wherein the means for sampling performs sampling in real time.

4. An ambulatory recorder according to any preceding claim wherein the second processor (22) has means for processing the data so the processed data may be graphically displayed to a user of the device.

5. An ambulatory recorder according to any preceding claim, wherein the first processor (21) requires a first amount of energy to perform the processing, and the second processor (22) requires a second amount of energy to perform the processing.

6. An ambulatory recorder according to claim 5 wherein the first amount of energy is less than the second amount of energy.

7. An ambulatory recorder according to any preceding claim further comprising means (10) for programming the first processor to process a programmed number of physiologic parameters from the patient.

8. An ambulatory recorder according to claim 7, wherein the first processor comprises said means (10) for programming the first processor to process a programmed number of physiologic parameters from the patient.

9. An ambulatory recorder according to claim 8 wherein the first processor (21) comprises means for sampling the sensed physiologic parameter.

10. An ambulatory recorder according to any preceding claim further comprising a mounting (74) for mounting the ambulatory recorder (1) to a patient.

11. An ambulatory recorder according to claim 10
wherein the mounting comprises a loop (74) configured for a belt or a shoulder strap to be inserted therethrough.

12. An ambulatory recorder according to any preceding claim wherein the sensor (2) comprises a pH sensing catheter.

## Patentansprüche

1. Ambulantes medizinisches Datenaufzeichnungsgerät zum Aufzeichnen wenigstens eines physiologischen Signals, das in einem Patienten durch einen Sensor erfasst wird, mit einem Gehäuse (55), das so beschaffen ist, dass es von einem Patienten getragen oder befördert werden kann, wobei das Aufzeichnungsgerät (55) enthält:
einen Sensor (2, 23), der mit dem Gehäuse gekoppelt ist, um das wenigstens eine erfasste physiologische Signal zu empfangen; wobei das Gehäuse enthält:
wenigstens eine Batterie (20), die eine Quelle für elektrischen Strom bereitstellt;
einen ersten Echtzeit-Prozessor (21), der mit dem Sensoreingang (23) operativ bzw. funktional verbunden ist und durch die wenigstens eine Batterie mit Leistung versorgt wird; und
einen zweiten Nichtechtzeit-Prozessor (22), der mit dem ersten Datenprozessor funktional verbunden ist und durch die wenigstens eine Batterie mit Leistung versorgt wird;
wobei während einer ersten Programmierungsphase der erste Prozessor ausgeschaltet ist und der zweite Prozessor mit Leistung versorgt wird bzw. ist und so konfiguriert ist, dass er Programmierungsinformationen empfängt;
während einer zweiten Datenabtastphase der erste Prozessor intermittierend in definierten vorgegebenen Zeitperioden, in denen er so konfiguriert ist, dass er wenigstens ein erfasstes physiologisches Signal von dem Sensoreingang empfängt und daran wenigstens einen ersten Prozess ausführt, um eine erste Datenmenge zu liefern und um diese in einem ersten Speicher zu speichern, eingeschaltet und aufgeweckt wird bzw. ist und der zweite Prozessor in regelmäßigen Intervallen, in denen er so konfiguriert ist, dass er die erste Datenmenge von dem ersten Prozessor empfängt und diese in einem zweiten Speicher speichert, aufgeweckt wird bzw. ist, und
während einer dritten Abfrage- und Datenherunterladephase der erste Prozessor ausgeschaltet und der zweite Prozessor eingeschaltet ist; und
während einer vierten Analysephase der erste und der zweite Prozessor ausgeschaltet sind.

2. Ambulantes Aufzeichnungsgerät nach Anspruch 1, bei dem der erste Prozessor Mittel (21) zum Abtasten des erfassten physiologischen Parameters umfasst.

3. Ambulantes Aufzeichnungsgerät nach Anspruch 2, bei dem die Mittel zum Abtasten die Abtastung in Echtzeit ausführen.

4. Ambulantes Aufzeichnungsgerät nach einem vorhergehenden Anspruch, bei dem der zweite Prozessor (22) Mittel besitzt, um die Daten zu verarbeiten, damit die verarbeiteten Daten für einen Anwender der Vorrichtung graphisch angezeigt werden können.

5. Ambulantes Aufzeichnungsgerät nach einem vorhergehenden Anspruch, bei dem der erste Prozessor (21) eine erste Energiemenge erfordert, um die Verarbeitung auszuführen, und der zweite Prozessor (22) eine zweite Energiemenge erfordert, um die Verarbeitung auszuführen.

6. Ambulantes Aufzeichnungsgerät nach Anspruch 5, bei dem die erste Energiemenge geringer als die zweite Energiemenge ist.

7. Ambulantes Aufzeichnungsgerät nach einem vorhergehenden Anspruch, das ferner Mittel (10) umfasst, um den ersten Prozessor so zu programmieren, dass er eine programmierte Anzahl physiologischer Parameter von dem Patienten verarbeitet.

8. Ambulantes Aufzeichnungsgerät nach Anspruch 7, bei dem der erste Prozessor die Mittel (10) zum Programmieren des ersten Prozessors umfasst, damit er eine programmierte Anzahl physiologischer Parameter von dem Patienten verarbeitet.

9. Ambulantes Aufzeichnungsgerät nach Anspruch 8, bei dem der erste Prozessor (21) Mittel umfasst, um den erfassten physiologischen Parameter abzutasten.

10. Ambulantes Aufzeichnungsgerät nach einem vorhergehenden Anspruch, das ferner eine Halterung (74) für die Anbringung des ambulanten Aufzeichnungsgeräts (1) an einem Patienten umfasst.

11. Ambulantes Aufzeichnungsgerät nach Anspruch 10, bei dem die Halterung eine Schlaufe (24) aufweist, die so konfiguriert ist, dass durch sie ein Riemen oder ein Schultergurt geführt werden kann.

12. Ambulantes Aufzeichnungsgerät nach einem vorhergehenden Anspruch, bei dem der Sensor (2) einen pH-Wert-Erfassungskatheter aufweist.

## Revendications

1. Dispositif d'enregistrement de données médicales ambulatoire pour enregistrer au moins un signal physiologique détecté chez un patient par un capteur, comportant un boîtier (55) adapté pour être porté ou transporté par un patient, le dispositif d'enregistrement (55) contenant :
un capteur (2, 23) couplé audit boîtier, pour recevoir le au moins un signal physiologique détecté, et le boîtier contenant :
au moins une batterie (20) pour fournir une source de courant électrique,
un premier processeur temps réel (21) relié opérationnellement à l'entrée de capteur (23) et alimenté par la au moins une batterie, et
un second processeur temps non-réel (22) relié opérationnellement au premier processeur de données et alimenté par la au moins une batterie,
dans lequel, durant une première phase de programmation, ledit premier processeur est désactivé et ledit second processeur est alimenté et configuré pour recevoir des informations de programmation,
durant une deuxième phase d'échantillonnage de données, ledit premier processeur est mis en marche et réveillé d'une intermittente à des périodes de temps prédéterminées définies durant lesquelles ledit premier processeur est configuré pour recevoir au moins un signal physiologique détecté depuis l'entrée de capteur et effectuer au moins un traitement sur celui-ci pour produire un premier ensemble de données et mémoriser celui-ci dans une première mémoire, et le second processeur est réveillé dans des intervalles réguliers durant lesquels ledit second processeur est configuré pour recevoir le premier ensemble de données à partir du premier processeur, et pour mémoriser celui-ci dans une seconde mémoire, et dans lequel :
durant une troisième phase d'interrogation et de téléchargement de données, ledit premier processeur est désactivé et ledit second processeur est alimenté, et
durant une quatrième phase d'analyse, les premier et second processeurs sont tous deux désactivés.

2. Dispositif d'enregistrement ambulatoire selon la revendication 1, dans lequel le premier processeur comporte des moyens (21) pour échantillonner le paramètre physiologique détecté.

3. Dispositif d'enregistrement ambulatoire selon la revendication 2, dans lequel les moyens d'échantillonnage effectuent un échantillonnage en temps réel.

4. Dispositif d'enregistrement ambulatoire selon l'une quelconque des revendications précédentes, dans lequel le second processeur (22) a des moyens pour traiter les données de manière à ce que les données traitées puissent être graphiquement affichées à un utilisateur du dispositif.

5. Dispositif d'enregistrement ambulatoire selon l'une quelconque des revendications précédentes, dans lequel le premier processeur (21) requiert une première quantité d'énergie pour effectuer le traitement, et le second processeur (22) requiert une seconde quantité d'énergie pour effectuer le traitement.

6. Dispositif d'enregistrement ambulatoire selon la revendication 5, dans lequel la première quantité d'énergie est inférieure à la seconde quantité d'énergie.

7. Dispositif d'enregistrement ambulatoire selon l'une quelconque des revendications précédentes, comportant de plus des moyens (10) pour programmer le premier processeur pour traiter un nombre programmé de paramètres physiologiques du patient.

8. Dispositif d'enregistrement ambulatoire selon la revendication 7, dans lequel le premier processeur comporte lesdits moyens (10) pour programmer le premier processeur pour traiter un nombre programmé de paramètres physiologiques du patient.

9. Dispositif d'enregistrement ambulatoire selon la revendication 8, dans lequel le premier processeur (21) comporte des moyens pour échantillonner le paramètre physiologique détecté.

10. Dispositif d'enregistrement ambulatoire selon l'une quelconque des revendications précédentes comportant de plus une monture (74) pour monter le dispositif d'enregistrement ambulatoire (1) sur un patient.

11. Dispositif d'enregistrement ambulatoire selon la revendication 10, dans lequel la monture comporte une boucle (74) configurée de manière à insérer à travers celle-ci une ceinture ou une bretelle.

12. Dispositif d'enregistrement ambulatoire selon l'une quelconque des revendications précédentes, dans lequel le capteur (2) comporte un cathéter de détection de pH.
